# EUROPÄISCHE PATENTSCHRIFT

(11) **EP 0 672 411 B1**
(45) Veröffentlichungstag und Bekanntmachung des Hinweises auf die Patenterteilung: **29.04.1998**
(21) Anmeldenummer: 94250276.6
(22) Anmeldetag: 15.11.1994
(51) Int. Cl.: A61K 31/135

(54) **Verwendung von 2-Methylamino-2-phenylcyclohexanon zur Behandlung von Infektionen und zur Immunmodulation**
Use of 2-methylamino-2-phenylcyclohexanon for the treatment of infections and as immunomodulator
Utilisation du 2-méthylamino-2-phényline hexanone pour le traitement des infections et comme immunomodulateur

(30) Priorität: 15.03.1994 DE 4409671
(43) Veröffentlichungstag der Anmeldung: 20.09.1995
(73) Patentinhaber: Preiss, Detlef, Dr., 14482 Potsdam (DE)
(72) Erfinder: Preiss, Detlef, Dr., D-14482 Potsdam (DE); Tatar, Akos, D-10963 Berlin (DE)
(74) Vertreter: Wablat, Wolfgang, Dr.Dr.

(56) Entgegenhaltungen:
- US-A- 3 254 124
- CHEMICAL ABSTRACTS, vol. 77, no. 17, 23.Oktober 1972 Columbus, Ohio, US; abstract no. 114046g, & JP-A-47 022 816

## Beschreibung

Die Erfindung betrifft die Verwendung von 2-Methylamino-2-phenylcyclohexanon (MPCH) oder dessen physiologisch verträglichen Salzen zur Herstellung von Arzneimitteln zur Behandlung von Bakterien-, Pilz-, Virus- oder Protozoen-Infektionen und zur Immunmodulation.

Bei der Behandlung von Infektionen werden in der heutigen Medizin eine große Zahl pharmazeutischer Wirkstoffe verwendet. Diese Infektionen gehen häufig mit Beeinträchtigungen des Immunsystems einher und werden auf der anderen Seite durch ein geschwächtes Immunsystem begünstigt.

Für das Gebiet der Bakterien- und Pilzinfektionen stehen eine Reihe sogenannter Breitbandtherapeutika zur Verfügung, welche gegen eine Vielzahl von unterschiedlichen Bakterienstämmen oder Pilzkulturen wirksam sind.

Im Bereich der Virusinfektionen sind dagegen nur wenige Wirkstoffe bekannt, die ihrerseits nur spezifisch gegen ganz bestimmte Virusstämme wirksam sind.

Ein ähnliches Bild ergibt sich bei den durch Protozoen hervorgerufenen Infektionen. Auch hier stehen nur spezifisch wirksame Therapeutika zur Verfügung, welche jeweils auf einen ganz bestimmten Erregertyp ansprechen.

Da Infektionserkrankungen häufig mit einem beschleunigten Verlauf der Erkrankung einhergehen, da die Vermehrung der Erreger in exponentiellem Maße erfolgt, ist es wünschenswert, Therapeutika zur Verfügung zu haben, welche aufgrund ihrer breiten Wirkung als Mittel der Wahl für die Erstmedikation verwendet werden können.

Diese Forderung ist zur Zeit nur auf dem Gebiet der bakteriellen und Pilzinfektionen in einem hinreichenden Umfang gewährleistet. Auf dem Gebiet der viralen und Protozoen-Infektion gibt es dagegen solche Breitbandtherapeutika bis heute nicht.

Aufgabe der vorliegenden Erfindung ist es daher, einen Wirkstoff zur Verfügung zu stellen, welcher zur Herstellung von Arzneimitteln zur Behandlung einer großen Zahl von durch verschiedene Bakterien, Pilze, Viren oder Protozoen hervorgerufenen Infektionen und zur Immunmodulation geeignet ist. Die entsprechenden Arzneimittel sollen durch eine geringe Toxizität, gute Verträglichkeit und hohe therapeutische Breite gekennzeichnet sein.

Diese Aufgabe wird erfindungsgemäß durch die Verwendung von 2-Methylamino-2-phenylcyclohexanon (MPCH) oder dessen physiologisch verträglichen Salzen zur Herstellung von Arzneimitteln zur Behandlung von Bakterien-, Pilz-, Virus- oder Protozoen-Infektionen und zur Immunmodulation gelöst.

Überraschenderweise wurde gefunden, daß 2-Methylamino-2-phenylcyclohexanon bei einer großen Anzahl bakterieller, fungischer, viraler und protozoaler Erkrankungen und zur Immunmodulation wirksam ist.

2-Methylamino-2-phenylcyclohexanon (MPCH) ist aus der U.S.-PS 3,254,124 bekannt. Darin wird MPCH als Verbindung mit kataleptischer Aktivität beschrieben.

Chemical Abstracts, vol.77 No. 17 (1972), Abstract No. 114046g beschreibt die Verwendung von MPCH als anästhetisches oder schmerzstillendes Produkt.

Überraschenderweise hat sich gezeigt, daß MPCH gegen eine Reihe unterschiedlicher Herpesviren wirkt. Es konnte gezeigt werden, daß MPCH sowohl gegen Herpes labialis, Herpes genitalis, Herpes zoster und Herpes simplex wirksam ist. Weiterhin konnte die Wirksamkeit gegen Zytomegalie-Viren (CMV) und gegen HIV-Viren nachgewiesen werden.

Insbesondere bei der Behandlung der HIV-Infektion hat sich überraschenderweise gezeigt, daß die mit der HIV-Infektion einhergehenden opportunistischen Infektionen in hohen Maße gleichfalls zurückgedrängt werden. Es ist bekannt, daß HIV-infizierte Personen häufig an Angina Plaut vinccentii, Candidiasis, Zytomegalie, Pneumocystien und diversen Herpes-Infektionen leiden. Diese Begleitinfektionen ließen sich durch die Behandlung mit MPCH gleichfalls zurückdrängen, so daß der Zustand des HIV infizierten Patienten in dramatischer Weise gebessert werden konnte.

Bei der Verwendung von 2-Methylamino-2-phenylcyclohexanon (MPCH) zur Herstellung von Arzneimitteln zur Behandlung von Bakterien-, Pilz-, Virus- oder Protozoen-Infektionen und zur Immunmodulation wird 2-Methylamino-2-phenylcyclohexanon gegebenenfalls in ein Säureadditionssalz, vorzugsweise in ein Salz einer physiologisch veträglichen Säure überführt.

Übliche physiologisch verträgliche anorganische und organische Säuren sind beispielsweise Salzsäure, Bromwasserstoffsäure, Phosphorsäure, Schwefelsäure, Oxalsäure, Maleinsäure, Fumarsäure, Milchsäure, Weinsäure, Äpfelsäure, Citronensäure, Salicylsäure, Adipinsäure und Benzoesäure. Weitere verwendbare Säuren sind beispielsweise in Fortschritte der Arzneimittelforschung, Bd. 10, Seiten 224-225, Birkhäuser Verlag, Basel und Stuttgart, 1966, und Journal of Pharmaceutical Sciences, Bd. 66, Seiten 1-5 (1977) beschrieben.

Die Säureadditionssalze werden in der Regel in an sich bekannter Weise durch Mischen der freien Base oder deren Lösungen mit der entsprechenden Säure oder deren Lösungen in einem organischen Lösungsmittel, beispielsweise einem niederen Alkohol wie Methanol, Ethanol, n-Propanol oder Isopropanol oder einem niederen Keton wie Aceton, Methylethylketon oder Methyl-isobutylketon oder einem Ether wie Diethylether, Tetrahydrofuran oder Dioxan, erhalten. Zur besseren Kristallabscheidung können auch Mischungen der genannten Lösungsmittel verwendet werden. Darüber hinaus können physiologisch verträgliche wäßrige Lösungen von Säureadditionssalzen von MPCH in einer wäßrigen Säurelösung hergestellt werden.

Die Säureadditionssalze von MPCH können in an sich bekannter Weise, z. B. mit Alkalien oder Ionenaustauschern, in die freie Base überführt werden. Von der freien Base lassen sich durch Umsetzung mit anorganischen oder organischen Säuren, insbesondere solchen, die zur Bildung von therapeutisch verwendbaren Salzen geeignet sind, weitere Salze gewinnen. Diese oder auch andere Salze der neuen Verbindung, wie z. B. das Pikrat, können auch zur Reinigung der freien Base dienen, indem man die freie Base in ein Salz überführt, dieses abtrennt und aus dem Salz wiederum die Base freisetzt.

Die Arzneimittel der Erfindung werden mit den üblichen festen oder flüssigen Trägerstoffen oder Verdünnungsmitteln und den üblicherweise verwendeten pharmazeutischtechnischen Hilfsstoffen entsprechend der gewünschten Applikationsart mit einer geeigneten Dosierung in bekannter Weise hergestellt. Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver, Lösungen oder Suspensionen oder Depotformen.

Selbstverständlich kommen auch parenterale Zubereitungen wie Injektionslösungen in Betracht. Weiterhin seien als Zubereitungen beispielsweise auch Suppositorien genannt.

Entsprechende Tabletten können beispielsweise durch Mischen des Wirkstoffs mit bekannten Hilfsstoffen, beispielsweise inerten Verdünnungsmitteln wie Dextrose, Zucker, Sorbit, Mannit, Polyvinylpyrrolidon, Sprengmitteln wie Maisstärke oder Alginsäure, Bindemitteln wie Stärke oder Gelantine, Gleitmitteln wie Magnesiumstearat oder Talk und/oder Mitteln zur Erzielung eines Depoteffektes wie Carboxylpolymethylen, Carboxylmethylcellulose, Celluloseacetatphthalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummiarabicum, Talk, Titandioxid oder Zucker, hergestellt werden. Dabei kann auch die Drageehülle aus mehreren Schichten bestehen, wobei die oben bei den Tabletten erwähnten Hilfsstoffe verwendet werden können.

Lösungen oder Suspensionen mit dem erfindungsgemäßen Wirkstoff können zusätzlich geschmacksverbessernde Mittel wie Saccharin, Cyclamat oder Zucker sowie z. B. Aromastoffe wie Vanillin oder Orangenextrakt enthalten. Sie können außerdem Suspendierhilfsstoffe wie Natriumcarboxymethylcellulose oder Konservierungsstoffe wie p-Hydroxybenzoate enthalten. Wirkstoffe enthaltende Kapseln können beispielsweise hergestellt werden, indem man den Wirkstoff mit einem inerten Träger wie Milchzucker oder Sorbit mischt und in Gelatinekapseln einkapselt.

Geeignete Suppositorien lassen sich beispielsweise durch Vermischen mit dafür vorgesehenen Trägermitteln wie Neutralfetten oder Polyäthylenglykol bzw. deren Derivaten herstellen.

Folgende Beispiele erläutern die Erfindung:

### Beispiel 1:

### Darstellung von 2-Methylamino-2-phenyl-cyclohexanon-hydrochlorid

Stufe 1:
   Zu einer Lösung von 14 g (80 mmol) Cyclopentylketon in 200 ml wasserfreiem Ether werden unter Rühren 14 g (196 mmol) Brom getropft. Danach wird die Lösung 30 Minuten unter Rückfluß erhitzt. Das Lösemittel wird unter vermindertem Druck entfernt, das verbleibende gelbe Öl in 20 ml Petrolether gelöst und zur Kristallisation gebracht.
Ausbeute:
   14 g (69 % d. Th.) 1-Benzoyl-1-Bromcyclopentan
Fp.: 28-30 °C
Stufe 2:
   12 g (47 mmol) 1-Benzoyl-1-Bromcyclopentan werden bei -20 °C mit 30 ml flüssigem Methylamin versetzt. Man läßt das Reaktionsgemisch innerhalb einer Stunde auf Raumtemperatur erwärmen. Nach Zusatz von 50 ml Ether wird das gebildete Salz abgesaugt, das Lösemittel unter vermindertem Druck und die verbleibende Kristalle getrocknet.
Ausbeute:
   2,95 g (31 % d. Th.) 1-Hydroxycyclopentylphenylketon-N-methylimin
Fp.: 72-74 °C
Stufe 3:
   2,95 g (14,5 mmol) 1-Hydroxycyclopentylphenylketon-N-methylimin wird in 30 ml Dekalin gelöst und 2 Stunden lang unter Rückfluß erhitzt. Nach dem Abkühlen wird das Reaktionsgemisch mit einem geringen molaren Überschuß an HCl-Gas gesättigtem 2-Propanol versetzt. Die Produkt werden abgesaugt und aus 2-Propanol/Ether umkristallisiert.
Ausbeute:
   3,3 g der Titelverbindung
Fp.: 255-257 °C

### Beispiel 2:

### Behandlung von Toxoplasmose

Ein Patient mit einer CT-gesicherten multifokalen cerebralen Toxoplasmose in einem schlechten Allgemeinzustand wurde wie folgt mit MPCH behandelt:
acht Wochen lang 2 mal wöchentlich mit jeweils 2 mg MPCH, danach vier Wochen lang keine Medikation und abschließend zwei Wochen lang 2 mal wöchentlich mit jeweils 2 mg MPCH.

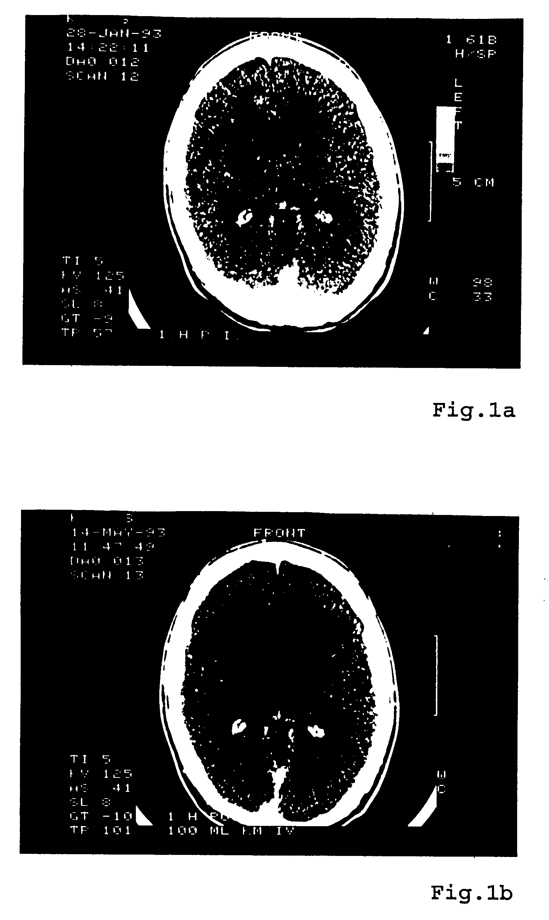

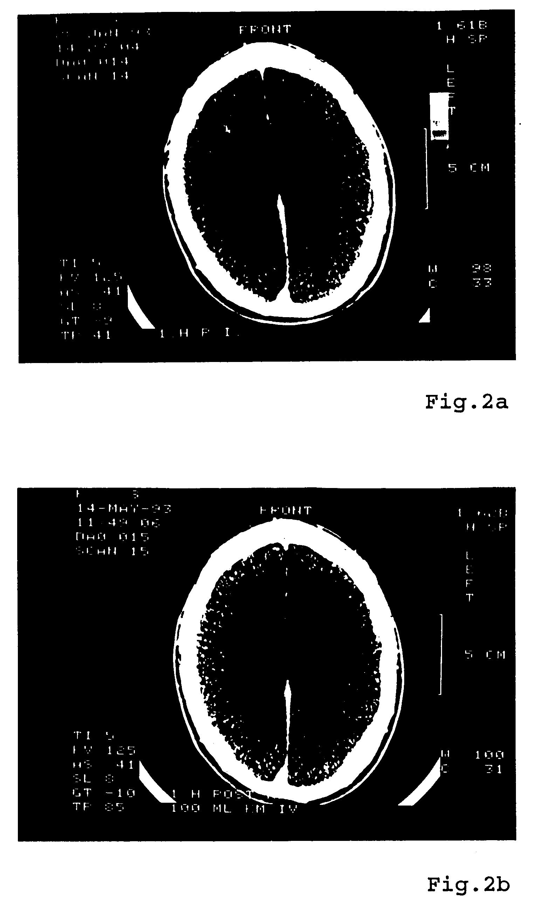

Der Allgemeinzustand des Patienten verbesserte sich bereits deutlich nach einem Monat. In Fig. 1a, 1b und Fig. 2a, 2b sind Kontrollaufnahmen des Cerebrums des Patienten mittels CT dargestellt, die den starken Rückgang von Toxoplasmoseherden nach der MPCH-Behandlung zeigen. Fig. 1a und Fig. 2a zeigen CT-Aufnahmen verschiedener Ebenen des Kopfes des Patienten vor der MPCH-Behandlung. In Fig. 1a und Fig. 2a sind jeweils im vorderen Drittel der linken Gehirnhälfte Toxoplasmoseherde erkennbar. Fig. 1b und Fig. 2b zeigen CT-Aufnahmen der entsprechenden Bereiche nach einer MPCH-Behandlung. Es ist eine deutliche bis vollkommene Rückbildung der Toxoplasmoseherde zu erkennen.

### Beispiel 3:

### Behandlung von Zytomegalievirus-Erkrankungen (CMV)

Ein Patient mit Fieber und schlechtem Allgemeinzustand und einer generalisierten Lymphknotenschwellung sowie einer CMV-Konjunktivitis (Serumtest) wurde am ersten, dritten, vierten und fünften Tag mit jeweils 2 mg MPCH behandelt. Am dritten Tag war der Patient fieberfrei, am vierten Tag waren die Lymphknotenschwellungen fast vollständig verschwunden, die Konjunktivitis am sechsten Tag bei gutem Allgemeinzustand.

### Beispiel 4:

### Behandlung von Herpes-Infektionen

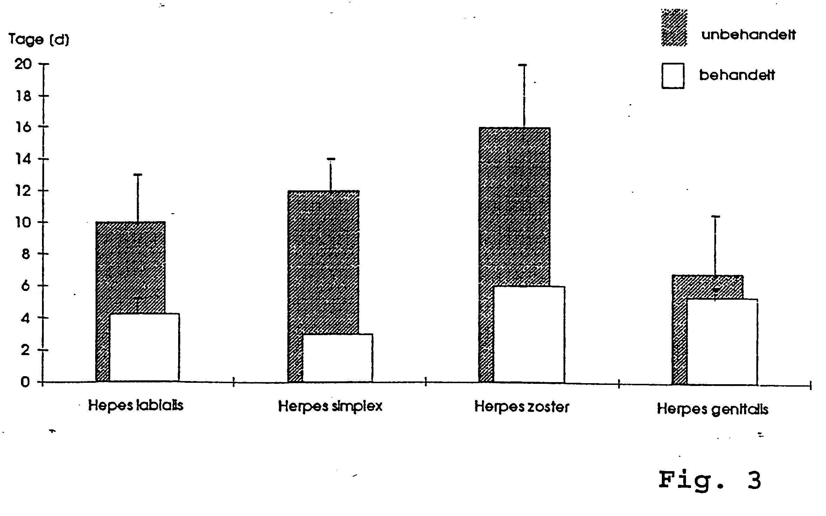

Es wurden verschiedene Patientengruppen mit Herpes labialis-, Herpes genitalis-, Herpes zoster- und Herpes simplex-Erkrankungen mit MPCH-Gaben behandelt. Die Patienten wurden jeweils 4-5 Tage lang mit jeweils 2 mg MPCH medikamentiert. Am Ende des Behandlungszeitraums waren die Erkrankungen in statistisch signifikanter Weise geheilt (vgl. Fig. 3).

### Beispiel 5:

### Behandlung von HIV-Erkrankungen

Ein seit über 10 Jahren HIV positiver Patient mit einer unspezifischen Mykoplasmeninfektion und Perimyokarditis in schlechtem Allgemeinzustand mit Neuropathien, wurde sechs Wochen lang 2 mal wöchentlich mit jeweils 2 mg MPCH behandelt. Dabei verbesserte sich der Allgemeinzustand deutlich, der Patient zeigte Gewichtszunahme und keine weiteren Infektionen.
Nach neun Monaten trat ein erneuter infektiöser Schub mit Angina Plaut vinccentii, Candidiasis, Zytomegalie sowie Herpes labialis auf. Der Patient wurde erneut sechs Wochen lang mit 2 mal wöchentlich 2 mg MPCH behandelt. Er erholte sich völlig von allen Infekten innerhalb des Behandlungszeitraumes.

## Patentansprüche

1. Verwendung von 2-Methylamino-2-phenylcyclohexanon (MPCH) oder dessen physiologisch verträglichen Salzen zur Herstellung von Arzneimitteln zur Behandlung von Bakterien-, Pilz-, Virus- oder Protozoen-Infektionen und zur Immunmodulation.

2. Verwendung gemäß Anspruch 1 von 2-Methylamino-2-phenylcyclohexanon oder dessen physiologisch verträglichen Salzen zur Behandlung von AIDS- und ARC (AIDS related complex)-Erkrankungen.

3. Verwendung gemäß Anspruch 1 von 2-Methylamino-2-phenylcyclohexanon oder dessen physiologisch verträglichen Salzen zur Behandlung von Herpes-Infektionen.

## Claims

1. Use of 2-methylamino-2-phenylcyclohexanone (MPCH) or its physiologically tolerable salts for the production of medicaments for the treatment of bacterial, fungal, viral or protozoan infections and for immunomodulation.

2. Use according to claim 1 of 2-methylamino-2-phenylcyclohexanone (MPCH) or its physiologically tolerable salts for the treatment of AIDS and ARC (AIDS related complex) diseases.

3. Use according to claim 1 of 2-methylamino-2-phenylcyclohexanone (MPCH) or its physiologically tolerable salts for the treatment of herpetic infections.

## Revendications

1. Utilisation de 2-méthylamino-2-phenylcyclohexanone (MPCH) ou de ses sels physiologiquement acceptables pour la préparation d'un médicament destiné au traitement des infections bactériennes, fongiques, virales ou par des protozoaires et pour l'immunomodulation.

2. Utilisation selon la revendication 1 de 2-méthylamino-2-phenylcyclohexanone ou de ses sels physiologiquement acceptables pour le traitement de maladies de type SIDA et ARC (AIDS related complex).

3. Utilisation selon la revendication 1 de 2-méthylamino-2-phenylcyclohexanone ou de ses sels physiologiquement acceptables pour le traitement des infections de type herpès.
